(19) Europäisches Patentamt
European Patent Office
Office européen des brevets

(11) Veröffentlichungsnummer: **0 222 205**
**B1**

(12) # EUROPÄISCHE PATENTSCHRIFT

(45) Veröffentlichungstag der Patentschrift: **14.09.88**

(51) Int. Cl.⁴: **C 07 D 233/56**

(21) Anmeldenummer: **86114457.4**

(22) Anmeldetag: **18.10.86**

(54) **Verfahren zur Herstellung von Imidazolyl-methan-Derivaten.**

(30) Priorität: **02.11.85 DE 3538873**

(43) Veröffentlichungstag der Anmeldung: **20.05.87 Patentblatt 87/21**

(45) Bekanntmachung des Hinweises auf die Patenterteilung: **14.09.88 Patentblatt 88/37**

(84) Benannte Vertragsstaaten: **AT BE CH DE ES FR GB IT LI NL SE**

(56) Entgegenhaltungen:
**METHODEN DER ORGANISCHEN CHEMIE, (HOUBEN-WEYL), vierte Auflage, Band XI/1, "Stickstoffverbindungen II", 1957, Seiten 643-650, Georg Thieme Verlag, Stuttgart, DE**

(73) Patentinhaber: **BAYER AG, Konzernverwaltung RP Patentabteilung, D-5090 Leverkusen 1 Bayerwerk (DE)**

(72) Erfinder: **Zerbes, Rudolf, Dr., In der Beek 26, D-5600 Wuppertal 1 (DE)**
Erfinder: **Regel, Erik, Dipl.-Ing., Untere Bergerheide 26, D-5600 Wuppertal 1 (DE)**

EP 0 222 205 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Herstellung von bekannten Imidazolyl-methan-Derivaten, welche antimykotische Eigenschaften besitzen.

Es ist bereits bekannt geworden, dass man Imidazolyl-methan-Derivate erhält, wenn man Carbinole entweder mit Thionyl-bis-imidazol umsetzt; oder zunächst in üblicher Weise halogeniert, und anschliessend mit Imidazol, gegebenenfalls in Gegenwart eines Säurebindemittels und in Gegenwart eines inerten organischen Lösungsmittels, umsetzt (vgl. hierzu DE-OS 2 461 406, DE-OS 2 130 673 und DE-OS 2 418 502).

Diese Verfahren haben entweder den Nachteil der Mehrstufigkeit (Keton-Derivat→Hydroxy-Derivat→Halogen-Derivat→Endprodukt) oder die Herstellung von Thionyl-bis-imidazol ist erforderlich. Beides führt zu unbefriedigenden Raum/Zeit-Ausbeuten bzw. zu hohen Herstellungskosten.

Ebenfalls ist bekannt, dass sich Amine mit Ketonen in Gegenwart von Ameisensäure in guter Ausbeute reduktiv alkylieren lassen (Leuckart-Wallach-Reaktion; vgl. hierzu z.B. Houben-Weyl, Methoden der Organischen Chemie, 4. Auflage, Band 11/1, S. 643 (1957); Methodicum Chimicum, Band 6, S. 536ff (1974); oder Org. Reactions *5*, 301 (1949). Entsprechende Umsetzungen mit ungesättigten Heterocyclen sind allerdings noch nicht beschrieben.

Es wurde nun gefunden, dass man die bekannten Imidazolyl-methan-Derivate der Formel (I)

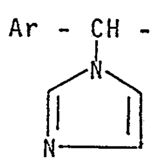

(I)

in welcher

Ar für gegebenenfalls einfach bis dreifach, gleich oder verschieden substituiertes Phenyl steht, wobei als Substituenten $C_1$-$C_6$-Alkyl, Halogen sowie jeweils gegebenenfalls durch Halogen oder $C_1$-$C_6$-Alkyl substituiertes Phenyl, Phenoxy und Phenylthio in Betracht kommen und

$R^1$ für geradkettiges oder verzweigtes Alkyl mit bis zu 6 Kohlenstoffatomen und für gegebenenfalls einfach bis dreifach, gleich oder verschieden substituiertes Phenyl steht, wobei als Substituenten Halogen, $C_1$-$C_6$-Alkyl und Halogen-$C_1$-$C_6$-Alkyl in Betracht kommen,

erhält, wenn man Ketone der Formel (II)

$$Ar - Co - R^1 \qquad (II)$$

in welcher

Ar und $R^1$ die oben angegebene Bedeutung haben,

mit Imidazol in Gegenwart von Ameisensäure umsetzt.

Es ist als überraschend zu bezeichnen, dass das erfindungsgemässe Verfahren die Verbindungen der Formel (I) in guten Ausbeuten liefert, da Imidazol kein Amin im engsten Sinn darstellt. Hinzu kommt die geringe Basizität des Imidazols im Vergleich zu den in der Literatur beschriebenen gesättigten heterocyclischen Aminen. Ausserdem stellt das Imidazol eine sterisch anspruchsvolle Verbindung dar, was bei der Reaktion mit Ketonen der Formel (II) mit ebenfalls sterisch anspruchsvollen Substituenten, wie z.B. Ar=Biphenyl und $R^1$=Phenyl, zu erheblichen Schwierigkeiten führen sollte. So sind Ketone vom Benzophenon Typ bei Leuckart-Wallach-Reaktionen auch bisher nicht eingesetzt worden.

Das erfindungsgemässe Verfahren zeichnet sich durch eine Reihe von Vorteilen aus. So handelt es sich um ein einfaches, einstufiges Verfahren, das zu guten Ausbeuten führt. Die als Ausgangsstoffe benötigten Verbindungen stellen billige Grundchemikalien dar.

Verwendet man beispielsweise 4-Benzoylbiphenyl als Ausgangsstoff, so kann der Verlauf des erfindungsgemässen Verfahrens durch das folgende Formelschema veranschaulicht werden:

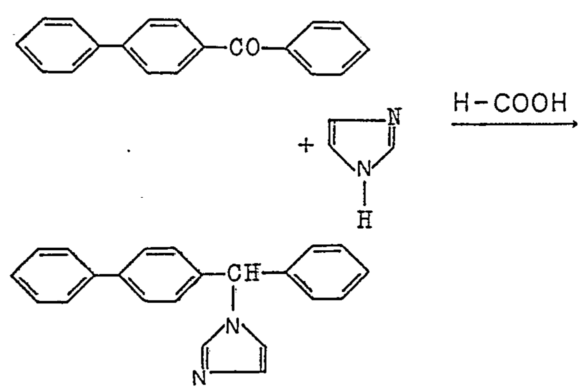


Die bei dem erfindungsgemässen Verfahren als Ausgangsstoffe zu verwendenden Ketone sind durch die Formel (II) allgemein definiert. In dieser Formel steht Ar vorzugsweise für gegebenenfalls einfach bis dreifach, gleich oder verschieden substituiertes Phenyl, wobei als Substituenten genannt seien: Alkyl, Halogen sowie jeweils gegebenenfalls durch Halogen oder Alkyl substituiertes Phenyl, Phenoxy und Phenylthio. $R^1$ steht vorzugsweise für geradkettiges oder verzweigtes Alkyl mit bis zu 6 Kohlenstoffatomen und für gegebenenfalls einfach bis dreifach, gleich oder verschieden substituiertes Phenyl, wobei als Substituenten genannt seien: Halogen, Alkyl sowie Halogenalkyl.

Besonders bevorzugte Ausgangsstoffe sind diejenigen Verbindungen der Formel (II), in denen

Ar für gegebenenfalls durch Fluor, Chlor, Methyl, Ethyl, Phenyl, Chlorphenyl, Phenoxy oder Chlorphenoxy substituiertes Phenyl steht und

$R^1$ für Methyl, Ethyl, n-Propyl, i-Propyl, n-Butyl, tert.-Butyl sowie für gegebenenfalls durch Fluor, Chlor, Methyl oder Trifluormethyl substituiertes Phenyl steht.

Ganz besonders bevorzugt sind Ausgangsstoffe der Formel (II), in denen

Ar für gegebenenfalls durch Chlor oder Phenyl substituiertes Phenyl steht und

$R^1$ für Methyl oder für gegebenenfalls durch Chlor substituiertes Phenyl steht.

Die Ketone der Formel (II) sind allgemein bekannte Verbindungen der organischen Chemie.

Die Reaktionstemperaturen können bei der Durchführung des erfindungsgemässen Verfahrens in einem grösseren Bereich variiert werden. Im allgemeinen arbeitet man bei Temperaturen zwischen 100° C und 250° C, vorzugsweise zwischen 150° C und 200° C.

Bei der Durchführung des erfindungsgemässen Verfahrens setzt man auf 1 Mol Keton der Formel (II) im allgemeinen 4 bis 12 Mol, vorzugsweise 6 bis 10 Mol Imidazol sowie 10 bis 50 Mol, vorzugsweise 20 bis 40 Mol Ameisensäure ein.

In manchen Fällen erweist es sich als vorteilhaft, in Gegenwart eines sauren Katalysators, wie beispielsweise Schwefelsäure oder p-Toluolsulfonsäure, zu arbeiten.

Die Isolierung der Endprodukte der Formel (I) erfolgt in allgemein üblicher und bekannter Art und Weise.

Die nach dem erfindungsgemässen Verfahren herstellbaren Imidazolyl-methan-Derivate der Formel (I) sind bekannt (vgl. Deutsche Offenlegungsschriften 2130673, 2418502 und 2461406). Sie zeichnen sich durch sehr gute antimykotische Eigenschaften aus.

Das erfindungsgemässe Verfahren wird durch die nachfolgenden Beispiele veranschaulicht.

*Beispiel 1*

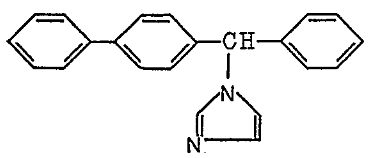

*Erfindungsgemäss*

64,5 g (0,25 Mol) 4-Benzoylbiphenyl, 136 g (2,0 Mol) Imidazol und 1,25 g p-Toluolsulfonsäure werden auf 180° C erhitzt. In 5 Stunden werden bei einer Reaktionstemperatur von 170-200° C insgesamt 368 g (8,0 Mol) Ameisensäure zugetropft. Während der Reaktion wird über eine Destillationsbrücke ein Gemisch aus Wasser und Ameisensäure (insgesamt 150 ml) destilliert. Nach dem Abkühlen auf 80° C werden zum Reaktionsgemisch 200 ml Toluol und 200 ml Wasser gegeben und 10 Minuten lang bei 80° C verrührt. Die Phasen werden getrennt und die organische Phase wird zweimal mit je 100 ml Wasser ausgerührt. Die 80° C Warme Toluol-Lösung wird mit 1 g Aktiv-Kohle und 3 g Kieselgur geklärt und im Vakuum zur Trockne eindampft.

Man erhält 56 g (72,3% der Theorie) 100%iges 4-Biphenyl-imidazol-1-yl-phenyl-methan vom Schmelzpunkt 142° C.

*Bekannt*

13,6 g (0,2 Mol) Imidazol werden in 150 ml Acetonitril gelöst und bei 10° C mit 3,5 ml Thionylchlorid versetzt. Zu der so erhaltenen Lösung von Thionyl-bis-imidazol gibt man 13 g (0,05 Mol) 4-Biphenyl-phenyl-carbinol. Nach 15-stündigem Stehen bei Raumtemperatur wird das Lösungsmittel durch Abdestillieren im Vakuum entfernt. Der Rückstand wird in Chloroform aufgenommen und mit Wasser gewaschen. Die organische Phase wird abgetrennt, über Natriumsulfat getrocknet, filtriert und das Lösungsmittel im Vakuum abdestilliert. Der ölige Rückstand wird im Essigester gelöst und durch Filtration von unlöslichen, harzigen Bestandteilen befreit. Das Lösungsmittel wird erneut im Vakuum abdestilliert und der Rückstand durch Umkristallisation aus Acetonitril gereinigt. Man erhält 8,7 g (56% der Theorie) 4-Biphenyl-imidazol-1-yl-phenylmethan vom Schmelzpunkt 142° C.

*Beispiel 2*

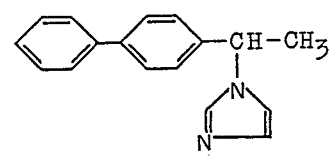

19,6 g (0,1 Mol) 4-Acetylbiphenylyl und 54,4 g (0,8 Mol) Imidazol werden auf 170° C bis 190° C erhitzt. Innerhalb von 2 Stunden werden 110,4 g (2,4 Mol) Ameisensäure zugetropft. Während der Reaktion wird über eine Destillationsbrücke ein Gemisch aus Wasser und Ameisensäure (ca. 50 ml) abdestilliert. Umsetzung (nach GC): 53,8% 2-(4-Biphenylyl)-2-imidazol-1-yl-ethan.

**Patentansprüche**

1. Verfahren zur Herstellung von Imidazolylmethan-Derivaten der allgemeinen Formel (I)

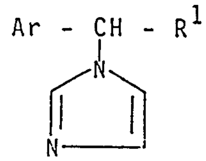

(I)

in welcher

Ar für gegebenenfalls einfach bis dreifach, gleich oder verschieden substituiertes Phenyl steht, wobei als Substituenten $C_1$-$C_6$-Alkyl, Halogen sowie jeweils gegebenenfalls durch Halogen oder $C_1$-$C_6$-Alkyl substituiertes Phenyl, Phenoxy und Phenylthio und Betracht kommen und

$R^1$ für geradkettiges oder verzweigtes Alkyl mit bis zu 6 Kohlenstoffatomen und für gegebenenfalls einfach bis dreifach, gleich oder verschieden substituiertes Phenyl steht, wobei als Substituenten Halogen, $C_1$-$C_6$-Alkyl und Halogen-$C_1$-$C_6$-alkyl in Betracht kommen,

dadurch gekennzeichnet, dass man Ketone der Formel (II)

$$Ar-CO-R^1 \qquad (II)$$

in welcher

Ar und $R^1$ die oben angegebene Bedeutung haben, mit Imidazol in Gegenwart von Ameisensäure

sowie gegebenenfalls in Anwesenheit eines sauren Katalysators umsetzt.

2. Verfahren gemäss Anspruch 1 zur Herstellung von Verbindungen der Formel (I), in denen

Ar für gegebenenfalls durch Fluor, Chlor, Methyl, Ethyl, Phenyl, Chlorphenyl, Phenoxy oder Chlorphenoxy substituiertes Phenyl steht und

$R^1$ für Methyl, Ethyl, n-Propyl, i-Propyl, n-Butyl, tert.-Butyl sowie für gegebenenfalls durch Fluor, Chlor, Methyl oder Trifluormethyl substituiertes Phenyl steht.

3. Verfahren gemäss Anspruch 1 zur Herstellung von Verbindungen der Formel (I), in denen

Ar für gegebenenfalls durch Chlor oder Phenyl substituiertes Phenyl steht und

$R^1$ für Methyl oder für gegebenenfalls durch Chlor substituiertes Phenyl steht.

4. Verfahren gemäss Ansprüchen 1-3, dadurch gekennzeichnet, dass man die Umsetzung bei Temperaturen zwischen 100 und 250° C durchführt.

5. Verfahren gemäss Ansprüchen 1-3, dadurch gekennzeichnet, dass man die Umsetzung bei Temperaturen zwischen 150 und 200° C durchführt.

6. Verfahren gemäss Ansprüchen 1-5, dadurch gekennzeichnet, dass man pro Mol Keton der Formel II 4-12 Mol Imidazol und 10-50 Mol Ameisensäure zusetzt.

7. Verfahren gemäss Ansprüchen 1-5, dadurch gekennzeichnet, dass man pro Mol Keton der Formel II 6-10 Mol Imidazol und 20-40 Mol Ameisensäure zusetzt.

## Claims

1. Process for the preparation of imidazolylmethane derivatives of the general formula (I)

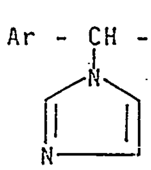

(I)

in which

Ar represents phenyl which is optionally mono-, di- or trisubstituted, identically or differently, suitable substituents being $C_1$-$C_6$-alkyl and halogen, and phenyl, phenoxy and phenylthio which are, in each case, optionally substituted by halogen or $C_1$-$C_6$-alkyl, and

$R^1$ represents straight-chain or branched alkyl having up to 6 carbon atoms and represents phenyl which is optionally mono-, di-or trisubstituted, identically or differently, suitable substituents being halogen, $C_1$-$C_6$-alkyl and halogeno-$C_1$-$C_6$-alkyl,

characterized in that ketones of the formula (II)

$$Ar-CO-R^1 \quad (II)$$

in which

Ar and $R^1$ have the abovementioned meaning, are reacted with imidazole in the presence of formic acid and, if appropriate, in the presence of an acidic catalyst.

2. Process according to Claim 1 for the preparation of compounds of the formula (I) in which

Ar represents phenyl which is optionally substituted by fluorine, chlorine, methyl, ethyl, phenyl, chlorphenyl, phenoxy or chlorophenoxy and

$R^1$ represents methyl, ethyl, n-propyl, i-propyl, n-butyl, tert.-butyl and phenyl which is optionally substituted by fluorine, chlorine, methyl or trifluoromethyl.

3. Process according to Claim 1 for the preparation of compounds of the formula (I), in which

Ar represents phenyl which is optionally substituted by chlorine or phenyl and

$R^1$ represents methyl, or phenyl which is optionally substituted by chlorine.

4. Process according to Claims 1-3, characterized in that the reaction is carried out at temperatures between 100 and 250° C.

5. Process according to Claims 1-3, characterized in that the reaction is carried out at temperatures between 150 and 200° C.

6. Process according to Claims 1-5, characterized in that 4-12 moles of imidazole and 10-50 moles of formic acid are added per mole of ketone of the formula II.

7. Process according to Claims 1-5, characterized in that 6-10 moles of imidazole and 20-40 moles of formic acid are added per mole of ketone of the formula II.

## Revendications

1. Procédé pour la fabrication de dérivés d'imidazolyl-méthane de formule générale (I)

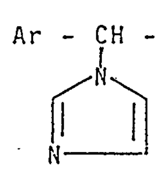

(I)

dans laquelle

Ar représente un radical phényle éventuellement substitué une à trois fois de façon identique ou différente, les substituants considérés étant alkyle $C_1$-$C_6$, halogène, ou phényle, phénoxy ou phénylthio éventuellement substitué chacun par halogène ou alkyle $C_1$-$C_6$, et

$R^1$ représente un groupe alkyle à chaîne droite ou ramifiée à 6 atomes de carbone maximum ou un phényle éventuellement substitué à une à trois fois de façon identique ou différente, les substituants entrant en ligne de compte étant halogène, alkyle $C_1$-$C_6$ ou alkyle $C_1$-$C_6$ halogéné,

caractérisé par le fait que l'on fait réagir des cétones de formule (II)

$$Ar-CO-R^1 \quad (II)$$

où

Ar et $R^1$ ont la même signification que ci-dessus, avec l'imidazole en présence d'acide formique et, le cas échéant, en présence d'un catalyseur acide.

2. Procédé selon la revendication 1 pour la fabrication de composés de formule (I), dans laquelle:

Ar représente un radical phényle éventuellement substitué par fluor, chlore, méthyle, éthyle, phényle, chlorophényle, phénoxy ou chlorophénoxy, et

$R^1$ représente un radical méthyle, éthyle, n-propyle, iso-propyle, n-butyle, butyle tertiaire ou phényle éventuellement substitué par fluor, chlore, méthyle ou trifluorométhyle.

3. Procédé selon la revendication 1 pour la fabrication de composés de formule (I) dans laquelle:

Ar représente un phényle éventuellement substitué par chlore ou phényle, et

$R^1$ représente un méthyle ou un phényle éventuellement substitué par chlore.

4. Procédé selon les revendications 1-3, caractérisé par le fait que l'on effectue la réaction à des températures comprises entre 100 et 250° C.

5. Procédé selon les revendications 1-3, caractérisé par le fait que l'on effectue la réaction à des températures comprises entre 150 et 200° C.

6. Procédé selon les revendications 1-5, caractérisé par le fait que l'on ajoute, par mole de cétone de formule II, 4-12 moles d'imidazole et 10-50 moles d'acide formique.

7. Procédé selon les revendications 1-5, caractérisé par le fait qu'on ajoute, par mole de cétone de formule II, 6-10 moles d'imidazole et 20-40 moles d'acide formique.